# EUROPEAN PATENT SPECIFICATION

(11) **EP 0 866 964 B1**
(45) Date of publication and mention of the grant of the patent: **24.01.2001**
(21) Application number: 96943955.3
(22) Date of filing: 06.12.1996
(51) Int. Cl.: G01N 33/32

(54) **COLOR TEST-PRINTING APPARATUS, ESPECIALLY FOR TEXTILE PRINTING WORKS**
FARBENTESTDRUCKGERAT, INSBESONDERE FUR TEXTILDRUCKVERFAHREN
APPAREIL D'IMPRESSION ET D'ECHANTILLONNAGE DE COULEURS DESTINE NOTAMMENT A DES TRAVAUX D'IMPRESSION DE TEXTILES

(30) Priority: 13.12.1995 IT MI952607
(43) Date of publication of application: 30.09.1998
(73) Proprietor: Somenzi, Enrica, 20152 Milano (IT)
(72) Inventor: Somenzi, Enrica, 20152 Milano (IT)
(74) Representative: De Gregori, Antonella
(86) International application number: EP9605612
(87) International publication number: WO9721998

(56) References cited:
- GB-A- 2 151 988
- GB-A- 2 204 279
- US-A- 3 926 112

## Description

This invention relates to a colour test-printing apparatus, in particular for textile printing works, ie a colour sampling apparatus.

The formation of new colours or the reproduction of already existing colours is one of the most complex operations of all production processes, particularly in textile printing works. In textile printing works, for example, this operation involves heavy commitment in terms of specialized human resources and in terms of operations and work to be performed to obtain a good quality result from the colour aspect. In this respect, a printed product is characterised by the multiple presence of different colours for the formation of its complex pattern. The colours normally reach a maximum of twentyfour for each of the variants of that pattern, which can range from one to ten or more. As can be seen a large number of samples and products are concerned, with a very rapid execution time.

By the use of spectrophotometry, modern technology has finally provided the designer with a fundamental instrument in article production. The facility for defining colour sensations in numerical terms and making them correspond to the average visual judgement has opened a horizon of new possible control and design techniques. The technique of colour-matching now enables the various components of colours to be identified with certainty, namely brightness, purity and tone. The formulation which achieves it (which takes account of the dyes, the light sources and other factors) is then used in the laboratory to print the test piece (sampling). The measurement has to be repeated with the spectrophotometer to verify the result obtained and calculate the possible new correction formula. This is repeated until the exactly sought colour formulation has been identified.

This is currently done in simple manual equipment in which a small quantity of colour is printed by hand on a portion of the support to be used, with successive verification of the result obtainable using the spectrophotometer. For this purpose a doctor blade for spreading the colour sample and a printing table are arranged in a suitable structure, between them there being positioned a piece of the support to be used. In such a sampling apparatus, in which the said colour sampling operation is carried out, each time a test or a sampling is carried out, the entire system, ie the doctor blade and printing table, has to be cleaned. Moreover this apparatus is an independent apparatus, not in line but positioned separately from the colour metering machine. There is hence currently a considerable manual intervention, with the continual cleanliness of the apparatus and its proper condition for repeating the test depending on the diligence and expertise of the user.

An object of the present invention is to provide a colour test-printing apparatus, in particular for textile printing works, which enables series of tests to be carried out without having to intervene each time for cleaning purposes.

A further object is to provide an apparatus which can be inserted into a test cycle and can be automated together with the colour manipulation and the movement of the test support for the colour being sampled.

These objects are attained according to the present invention by a colour test-printing apparatus, in particular for textile printing works, in accordance with claim 1. Further characteristics are the subject of the dependent claims.

The characteristics and advantages of a colour test-printing apparatus, in particular for textile printing works, formed in accordance with the present invention will be more apparent from the description thereof given hereinafter by way of non-limiting example with reference to the accompanying schematic drawings, in which:
Figure 1 is a perspective view of a colour test-printing apparatus, in particular for printing a textile support, according to the present invention;
Figure 2 is a view equal to that of Figure 1, with a portion of the apparatus in the open position;
Figure 3 is a side elevation of the apparatus of Figure 1;
Figure 4 is a plan view of the apparatus of Figure 1 taken from above.

The figures show one embodiment of a colour test-printing apparatus of the present invention for textile printing works, in a complex arrangement with which there is associated an automatic colour depositing unit.

In a more simplified arrangement, the apparatus can simple comprise a support structure 11a and 11b in two mutually separable parts, in the example hinged together along a longitudinal side, and between which there is passed a support 12 to be printed, for example a continuous support consisting of a fabric which unwinds upstream from an unwinding bobbin 13 and is rewound downstream onto another rewinding bobbin 14. The two portions 11a and 11b can be separated to locate a colour sample to be printed on the continuous support. This separation can for example be achieved by lifting one part from the other to enable the colour sample to be introduced.

A doctor blade 15 is located in the upper part 11a of the support structure, and in its lower part there is positioned a print table 16 which collaborates with the doctor blade to act on a region of the continuous support 12.

Both in the upper part 11a and in the lower part 11b of the support structure there are located further unwinding bobbins 17a and 17b and rewinding bobbins 18a and 18b for a continuous protection film 19 and 19', for example of plastics material, which embraces the continuous support 12 to be printed and prevents the colour, which is in contact with it and is spread, from coming into contact either with the doctor blade 15 or with the underlying printing table 16.

Associated with the support structure 11a and 11b and positioned downstream of it there is a spectrophotometer 20 which examines the printed region of the continuous support, checks it and determines any corrections to be made to the parameters.

Between the support structure 11a and 11b and the spectrophotometer 20 there is preferably positioned a heater 21 which facilitates a subsequent more reliable reading closer to the actual final result.

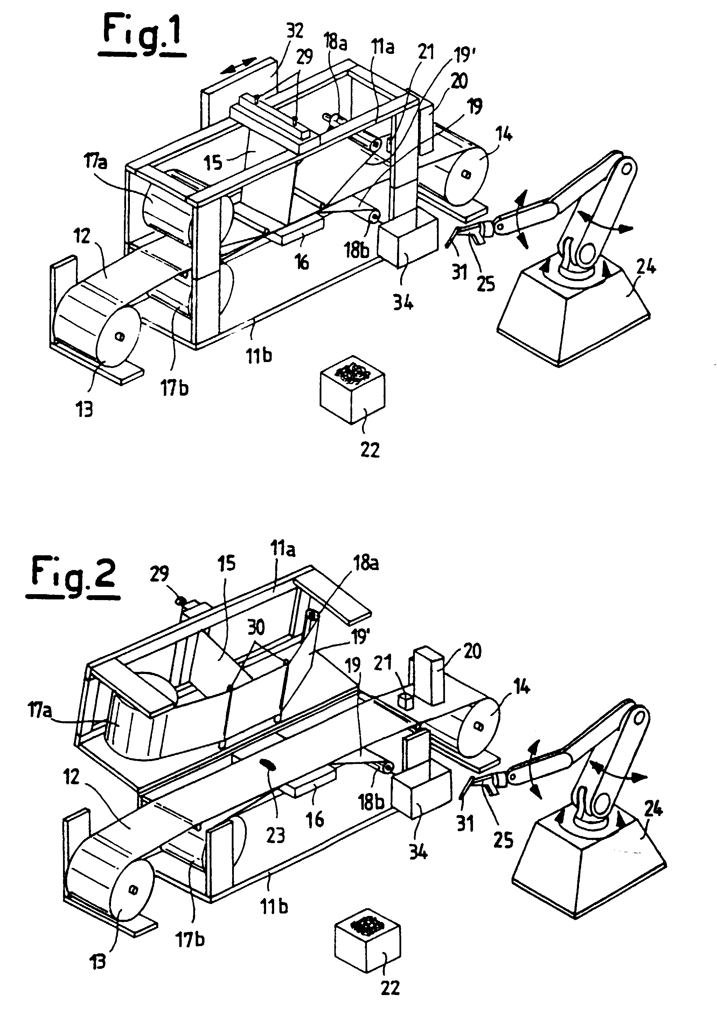

There is also provided an automatic colour withdrawal unit which, when the support is open as in Figure 2, withdraws a metered quantity of colour from a container or other system 22 and deposits it on the continuous support 12, as shown schematically at 23. This unit can consist of a robotized or motorized manipulator 24 with four degrees of freedom, provided with a gripper 25 able to grip a disposable withdrawal element 31 for the colour 23.

In the automated form a series of actuators for the various movements and operations must be provided, all connected to a central processor 26.

For example there must be provided at least one motor means 27 for advancing the continuous support 12 at variable speed so as to be able to simulate the various printing speeds. There must also be provided motor means 28 for unwinding the continuous protection films 19 and 19' stepwise according to the region on which the test-printing or colour sampling has been effected. Again, with the doctor blade 15 there can be associated means 29 for adjusting both its pressure and inclination so as to be able to simulate with major precision the actual printing conditions as the type of continuous support 12 varies. The spectrophotometer 20 must be associated with the processor 26 so as to follow the quantity and quality of readings, on the basis of the quality and type of continuous support 12 used and on the basis of the quantity of colour 23 positioned thereon. Deviator rollers 30 can be provided in the apparatus printing region to facilitate correct positioning of the two protection films 19 and 19', and also in the continuous support region 12 under test-printing. These deviator rollers 30 can be rigid for example with the upper part 11a of the support structure and act on the lower protection film 19 and on the continuous support only when the apparatus is closed.

As an alternative printing element to be associated with the printing table 16 instead of the doctor blade 15, a printing roller can simply be used without on this account changing the type of operation.

An apparatus of the present invention hence operates in the following manner.

Having selected the type of continuous support 12 to be used and the colours to be chosen, the containers are provided with the colour samples in proximity to the apparatus of the present invention.

The processor 26 is then set by providing it with all the data relative to the type of support 12, the type of colour used, the drying temperature, the number of passes, the advancement speed of the support 12 and the arrangement of the doctor blade or roller 15 (pressure and inclination). These data can also be supplied by another processor.

At this point the processor indicates on the basis of a predetermined program the functions to be performed and their correct succession.

The robotized manipulator 24 withdraws the colour sample 23 and, with the apparatus open as shown in Figure 2, deposits a spot of colour 23 on a region on the continuous support 12.

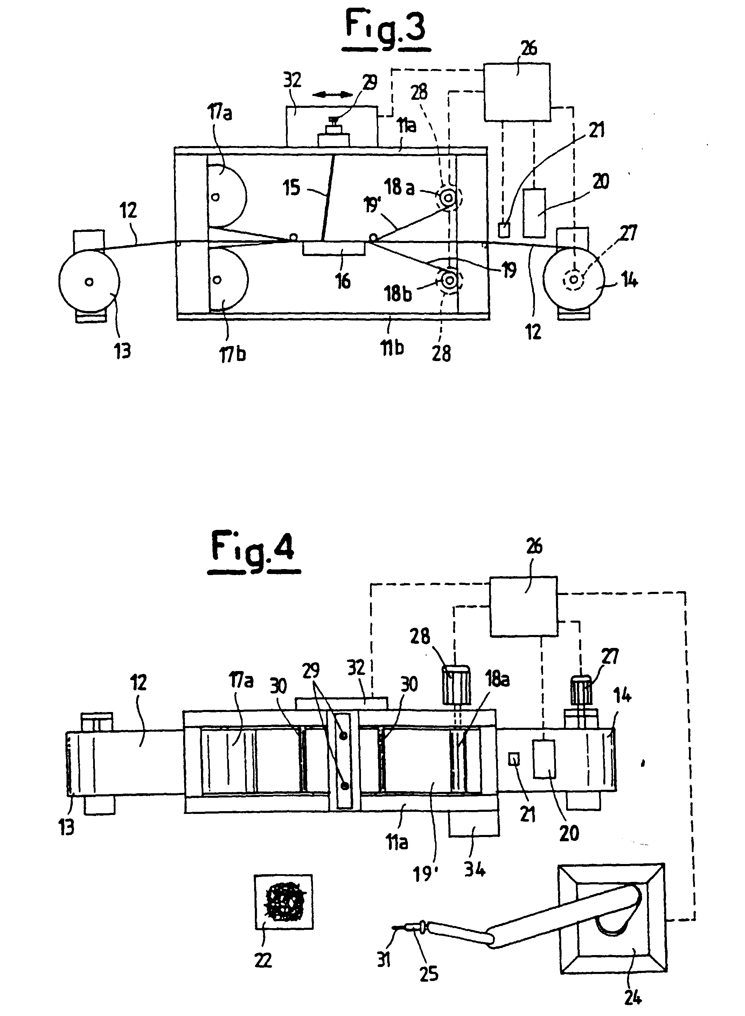

The apparatus is then closed as in Figures 1 and 3 and the motor means 32 is operated for advancing the printing element 15 with resultant printing of the continuous support 12.

During this printing operation the protective film 19' positioned between the doctor blade or roller 15 and the colour, and the protective film 19 positioned between the printing table 16 and the continuous support 12, prevent any contact between the colour and the parts of the apparatus, ensuring their final cleanliness. Cleanliness for test-printing or sampling purposes is immediately restored by operation of the motors 28, which advance the two protective films into a clean region and wind the soiled and used films onto the appropriate bobbins 18a and 18b.

This printed portion is made to advance by the motor means 27 to a successive downstream region where an appropriate heater 21 acts to dry the colour before the spectrophotometer reading.

The said reading allows any printed colour deficiencies to be identified and enables appropriate corrections to be made to the formulation for the next sampling test, which is carried out immediately once the colour container has been replaced with a new one.

All the printing tests are carried out without any cleaning being required.

## Claims

1. A colour test-printing apparatus, in particular for textile printing works, which comprises a support structure (11a,11b) and in which a metered quantity of colour (23) is positioned on a continuous support (12) and printed by a printing element (15) which collaborates with an underlying printing table (16) when a region of said continuous support (12) to be printed is interposed between them, characterised in that said support structure consists of two parts (11a,11b) separable in a direction away from each other for the introduction of said metered quantity of colour (23), said continuous support (12) being slidable below a continuous protection element (19'), which is unwound from and rewound on bobbins (17a,18a) and is interposed between said printing element (15) and said continuous support (12), downstream of said apparatus and associated with said support structure there being provided a spectrophotometer (20) for measuring a printed region of said continuous support (12).

2. An apparatus as claimed in claim 1, characterised by comprising a further continuous protection element (19) which unwinds from and rewinds onto further bobbins (17b,18b), said continuous protection elements (19,19') being mutually superposed at least in correspondence with said printing element (15) and said printing table (16), and being positioned externally on opposite sides of said continuous support (12).

3. An apparatus as claimed in claim 1 or 2, characterised in that said protection elements (19,19') consist of plastics films.

4. An apparatus as claimed in claim 1, characterised in that said two parts (11a, 11b) of the support structure are hinged together along one of their longitudinal sides.

5. An apparatus as claimed in claim 1, characterised by also comprising between said spectrophotometer (20) and said support structure (11a,11b) a heater (21) for said printed region (23).

6. An apparatus as claimed in claim 1, characterised in that said continuous support (12) unwinds upstream from an unwinding bobbin (13) and rewinds downstream onto another rewinding bobbin (14).

7. An apparatus as claimed in claim 1, characterised by having associated therewith a robotized or motorized manipulator (24) with four degrees of freedom, provided with a gripper (25) able to grip an element (31) for withdrawing a sample of colour from a respective container (22).

8. An apparatus as claimed in claim 1, characterised in that said printing element is a doctor blade (15).

9. An apparatus as claimed in claim 1, characterised in that said printing element is a roller.

10. An apparatus as claimed in claim 8 or 9, characterised in that said printing element is provided with elements (29) for adjusting the printing pressure.

11. An apparatus as claimed in claim 8, characterised in that said doctor blade is provided with elements (29) for adjusting its inclination.

12. An apparatus as claimed in claim 1, characterised in that in said region to be printed there are provided deviator rollers (30) which facilitate the correct positioning of at least one (19,19') of the continuous protection elements.

13. An apparatus as claimed in claim 12, characterised in that when the apparatus is closed, said deviator rollers (30) act on both said continuous protection elements (19,19'), and in said region of said continuous support (12) positioned above said printing table (16).

14. An apparatus as claimed in claim 7, characterised in that said withdrawal element (31) is of disposable type.

15. An apparatus as claimed in claim 2, characterised by having associated therewith a central processor (26) which has an operational cooperation with the motor means (28) for rotating said protection element (19,19') bobbins (18a,18b).

16. An apparatus as claimed in claim 6, characterised by having associated therewith a central processor (26) which has an operational cooperation with the motor means (27) for advancing said continuous support (12).

17. An apparatus as claimed in claim 7, characterised by having associated therewith a central processor (26) which coordinates the operational cooperation of said colour sample manipulator (24).

18. An apparatus as claimed in claim 5, characterised by having associated therewith a central processor (26) which coordinates the operational cooperation of said heater (21).

## Patentansprüche

1. Farbtestdruckvorrichtung, insbesondere für Textildruckverfahren, das ein Stützgerüst (11a, 11b) umfaßt, und bei dem eine abgemessene Farbmenge (23) auf einen fortlaufenden Träger (12) aufgebracht und von einem Druckelement (15), das mit einem darunterliegenden Drucktisch (16) zusammenwirkt, gedruckt wird, wenn ein Bereich des zu bedruckenden fortlaufenden Trägers (12) dazwischen angeordnet ist, dadurch **gekennzeichnet**, daß
das Stützgerüst aus zwei Teilen (11a, 11b) besteht, die zum Einführen der abgemessenen Farbmenge (23) voneinander trennbar sind,
der fortlaufende Träger (12) unter einem fortlaufenden Schutzelement (19') verschiebbar ist, das von Spulen (17a) ab- und auf Spulen (18a) aufgewickelt wird und zwischen dem Druckelement (15) und dem fortlaufenden Träger (12) angeordnet ist, und
in Laufrichtung und mit dem Stützgerüst verbunden ein Spektrophotometer (20) zum Messen eines bedruckten Bereiches des fortlaufenden Trägers (12) vorgesehen ist.

2. Vorrichtung nach Anspruch 1, ein weiteres fortlaufendes Schutzelement (19) umfassend, das von weiteren Spulen (17b) ab- und auf weitere Spulen (18b) aufgewickelt wird, wobei sich die fortlaufenden Schutzelemente (19, 19') zumindest im Bereich des Druckelements (15) und des Drucktischs (16) überlagern und jeweils außen auf gegenüberliegenden Seiten des fortlaufenden Trägers (12) angeordnet sind.

3. Vorrichtung nach Anspruch 1 oder 2, wobei die Schutzelemente (19, 19') aus Kunststoffolien bestehen.

4. Vorrichtung nach Anspruch 1, wobei die zwei Teile (11a, 11b) des Stützgerüsts entlang einer ihrer Längsseiten ein gemeinsames Scharnier haben.

5. Vorrichtung nach Anspruch 1, wobei zwischen dem Spektrophotometer (20) und dem Stützgerüst (11a, 11b) eine Heizvorrichtung (21) für den Druckbereich (23) vorhanden ist.

6. Vorrichtung nach Anspruch 1, wobei der fortlaufende Träger (12) von einer gegen die Laufrichtung angeordneten Abgabespule (13) ab- und auf eine in Laufrichtung angeordnete Aufnahmespule (14) aufgewickelt wird.

7. Vorrichtung nach Anspruch 1, mit einem roboterisierten oder motorisierten Manipulator (24) mit vier Freiheitsgraden zusammenhängend, der mit einem Greifer (25) versehen ist, der ein Element (31) zur Entnahme einer Farbprobe aus einem zugehörigen Behälter (22) aufnehmen kann.

8. Vorrichtung nach Anspruch 1, wobei das Druckelement eine Rakel (15) ist.

9. Vorrichtung nach Anspruch 1, wobei das Druckelement eine Walze ist.

10. Vorrichtung nach Anspruch 8 oder 9, wobei das Druckelement mit Elementen (29) zum Einstellen des Anpreßdrucks versehen ist.

11. Vorrichtung nach Anspruch 8, wobei die Rakel mit Elementen (29) zum Einstellen ihrer Schrägstellung versehen ist.

12. Vorrichtung nach Anspruch 1, wobei im Druckbereich Umlenkrollen (30) vorgesehen sind, die die richtige Positionierung mindestens eines der fortlaufenden Schutzelemente (19, 19') ermöglichen.

13. Vorrichtung nach Anspruch 12, wobei bei geschlossener Vorrichtung die Umlenkrollen (30) auf beiden Seiten der fortlaufenden Schutzelemente (19, 19') und in dem Bereich des fortlaufenden Trägers (12) wirken, der sich über dem Drucktisch (16) befindet.

14. Vorrichtung nach Anspruch 7, wobei das Entnahmeelement (31) ein Wegwerfteil ist.

15. Vorrichtung nach Anspruch 2, mit einem Zentralrechner 26 zusammenhängend, der mit der Motoranordnung (28) zum Antrieb der Schutzelement(19, 19')-Spulen (18a, 18b) zusammenwirkt.

16. Vorrichtung nach Anspruch 6, mit einem Zentralrechner (26) zusammenhängend, der mit der Motoranordnung (27) zum Antrieb des fortlaufenden Trägers (12) zusammenwirkt.

17. Vorrichtung nach Anspruch 7, mit einem Zentralrechner (26) zusammenhängend, der das Zusammenwirken mit dem Farbprobenmanipulator (24) koordiniert.

18. Vorrichtung nach Anspruch 5, mit einem Zentralrechner (26) zusammenhängend, der das Zusammenwirken mit der Heizeinrichtung (21) koordiniert.

## Revendications

1. Appareil pour essais d'impression en couleurs, destiné en particulier à des travaux d'impression sur textiles, qui comprend une structure de support (11a, 11b) et dans lequel une quantité mesurée de couleur (23) est placée sur un support continu (12) et imprimée par un élément d'impression (15) qui coopère avec une table d'impression sous-jacente (16) quand une zone dudit support continu (12) à imprimer est interposée entre ceux-ci, caractérisé en ce que ladite structure de support se compose de deux parties (11a, 11b) pouvant être séparées dans une direction les éloignant l'une de l'autre pour permettre l'introduction de ladite quantité mesurée de couleur (23), ledit support continu (12) pouvant coulisser sous un élément de protection continu (19'), qui est déroulé d'une bobine et enroulé sur une autre bobine (17a, 18a) et est interposé entre ledit élément d'impression (15) et ledit support continu (12), un spectrophotomètre (20) étant installé pour mesurer une zone imprimée dudit support continu (12), en aval dudit appareil, et associé à ladite structure de support.

2. Appareil selon la revendication 1, caractérisé en ce qu'il comprend un élément de protection continu supplémentaire (19) qui se déroule d'une bobine supplémentaire et s'enroule sur une autre bobine supplémentaire (17b, 18b), lesdits éléments de protection continus (19, 19') étant mutuellement superposés au moins en correspondance avec ledit élément d'impression (15) et ladite table d'impression (16) et étant placés à l'extérieur de part et d'autre dudit support continu (12).

3. Appareil selon la revendication 1 ou 2, caractérisé en ce que lesdits éléments de protection (19, 19') se composent de films en matière plastique.

4. Appareil selon la revendication 1, caractérisé en ce que lesdites deux parties (11a, 11b) de la structure de support sont articulées l'une sur l'autre le long d'un de leurs côtés longitudinaux.

5. Appareil selon la revendication 1, caractérisé en ce qu'il comprend également, entre ledit spectrophotomètre (20) et ladite structure de support (11a, 11b), un élément chauffant (21) destiné à ladite zone imprimée (23).

6. Appareil selon la revendication 1, caractérisé en ce que ledit support continu (12) se déroule en amont à partir d'une bobine de déroulement (13) et s'enroule en aval sur une autre bobine d'enroulement (14).

7. Appareil selon la revendication 1, caractérisé en ce qu'il comporte, associé à lui, un bras manipulateur robotisé ou motorisé (24) ayant quatre degrés de liberté, pourvu d'un préhenseur (25) capable de saisir un élément (31) pour prélever un échantillon de couleur à partir d'un récipient respectif (22).

8. Appareil selon la revendication 1, caractérisé en ce que ledit élément d'impression est une racle d'enduction (15).

9. Appareil selon la revendication 1, caractérisé en ce que ledit élément d'impression est un rouleau.

10. Appareil selon la revendication 8 ou 9, caractérisé en ce que ledit élément d'impression est pourvu d'éléments (29) destinés à régler la pression d'impression.

11. Appareil selon la revendication 8, caractérisé en ce que ladite racle d'enduction est pourvue d'éléments (29) destinés à régler son inclinaison.

12. Appareil selon la revendication 1, caractérisé en ce que, dans ladite zone à imprimer, sont ménagés des rouleaux de déviation (30) qui facilitent le positionnement correct d'au moins un des éléments de protection continus (19, 19').

13. Appareil selon la revendication 12, caractérisé en ce que, quand l'appareil est fermé, lesdits rouleaux de déviation (30) agissent sur lesdits deux éléments de protection continus (19, 19') et dans ladite zone dudit support continu (12) positionné au-dessus de ladite table d'impression (16).

14. Appareil selon la revendication 7, caractérisé en ce que ledit élément de prélèvement (31) est de type jetable.

15. Appareil selon la revendication 2, caractérisé en ce qu'il comporte, associé à lui, un processeur central (26) qui coopère de manière fonctionnelle avec les moyens moteurs (28) destinés à faire tourner lesdites bobines (18a, 18b) pour les éléments de protection (19, 19').

16. Appareil selon la revendication 6, caractérisé en ce qu'il comporte, associé à lui, un processeur central (26) qui coopère de manière fonctionnelle avec les moyens moteurs (27) destinés à faire avancer ledit support continu (12).

17. Appareil selon la revendication 7, caractérisé en ce qu'il comporte, associé à lui, un processeur central (26) qui coordonne la coopération fonctionnelle dudit manipulateur d'échantillons de couleur (24).

18. Appareil selon la revendication 5, caractérisé en ce qu'il comporte, associé à lui, un processeur central (26) qui coordonne la coopération fonctionnelle dudit élément chauffant (21).
